# EUROPEAN PATENT APPLICATION

(11) **EP 2 716 627 A1**
(43) Date of publication of application: **09.04.2014**
(21) Application number: 12792264.9
(22) Date of filing: 30.05.2012
(51) Int. Cl.: C07C 243/36, C07B 61/00, C07C 241/04, C07C 243/22, C07D 261/04

(54) **METHOD FOR PRODUCING ISOXAZOLINE COMPOUND**

(30) Priority: 03.06.2011 JP 2011125013; 31.01.2012 JP 2012017678
(71) Applicant: Sumitomo Chemical Company, Limited, Tokyo 104-8260 (JP)
(72) Inventor: UJIHARA, Kazuya, Takarazuka-shi Hyogo 665-8555 (JP); KUMAMOTO, Koji, Oaza Misawa, Misawa-shi Aomori 033-0022 (JP)
(74) Representative: Vossius & Partner
(86) International application number: PCT/JP2012/064490
(87) International publication number: WO 2012/165652

(57) **Abstract**

Provided are a novel method for producing an isoxazoline compound represented by formula (8): 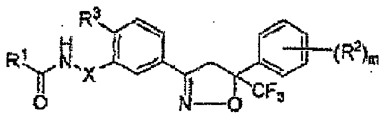 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, R² represents a C1-C6 alkyl group optionally having one or more halogen atoms or a halogen atom, R³ represents a halogen atom or a hydrogen atom, X represents NH or NCH₃, and m represents an integer of 0 to 5 (when m is an integer of 2 to 5, R²s may be different from one another)]
and an intermediate for the production of the isoxazoline compound.

## Description

### TECHNICAL FIELD

The present invention relates to a method for producing an isoxazoline compound and a production intermediate thereof.

### BACKGROUND ART

It has been known that an isoxazoline compound represented by the following formula (8) is useful, for example, as an active ingredient of a pesticide. In addition, for example, WO2010/032437 discloses a method for synthesizing an isoxazoline compound represented by the following formula (8).

### DISCLOSURE OF THE INVENTION

The above conventional method is not necessarily satisfactory in the production of the isoxazoline compound represented by the following formula (8).

Therefore, an object of the present invention is to provide a new method that can produce the isoxazoline compound represented by the following formula (8) and a production intermediate thereof.

As a result of intensive studies, the present inventors have found that an isoxazoline compound represented by the following formula (8) can be produced by
reacting a hydrazine compound represented by the following formula (1) with an acid anhydride compound represented by the following formula (2) or an acid chloride represented by formula (3) to produce a hydrazide compound represented by the following formula (4),
reacting the obtained hydrazide compound represented by the following formula (4) with a trifluoroacetophenone compound represented by the following formula (5) in the presence of a base to produce an aldol compound represented by the following formula (6),
reacting the obtained aldol compound represented by the following formula (6) with the acid anhydride compound represented by the following formula (2) and then reacting the resultant with a base to produce an enone compound represented by the following formula (7), and
reacting the obtained enone compound represented by the following formula (7) with hydroxylamine.

More specifically, the present invention is as described below.
[1] A hydrazide compound represented by formula (4): 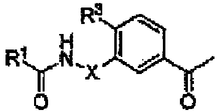 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, R³ represents a halogen atom or a hydrogen atom, and X represents NH or NCH₃].
[2] An aldol compound represented by formula (6) : 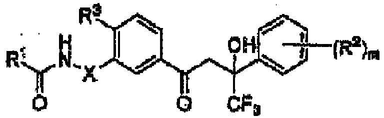 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, R² represents a C1-C6 alkyl group optionally having one or more halogen atoms or a halogen atom, R³ represents a halogen atom or a hydrogen atom, X represents NH or NCH₃, and m represents an integer of 0 to 5 (when m is an integer of 2 to 5, R²s may be different from one anther)]
[3] (3-Acetyl-6-chlorophenyl)hydrazine.
[4] A method for producing a hydrazide compound represented by formula (4): 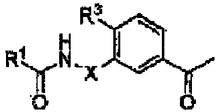 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, R³ represents a halogen atom or a hydrogen atoms, and X represents NH or NCH₃] [hereinafter referred to as hydrazide compound (4)], comprising a step of reacting a hydrazine compound represented by formula (1): 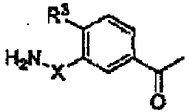 [wherein R³ represents a halogen atom or a hydrogen atom, and X represents NH or NCH₃] (hereinafter referred to as hydrazine compound (1)) with an acid anhydride compound represented by formula (2): 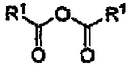 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms] (hereinafter referred to as acid anhydride compound (2)) or an acid chloride represented by formula (3): 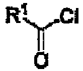 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms] (hereinafter referred to as acid chloride (3)).
[5] A method for producing an aldol compound represented by formula (6): 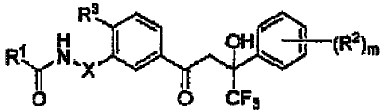 [wherein R¹ represents a C1-C12 alkyl group optionally having one or more halogen atoms, a C3-C12 cycloalkyl group optionally having one or more halogen atoms or a C1-C12 alkoxy group optionally having one or more halogen, atoms, R² represents a C1-C6 alkyl group optionally having one or more halogen atoms, or a halogen atom, R³ represents a halogen atom or a hydrogen atom, X represents NH or NCH₃, and m represents an integer of 0 to 5 (when m is an integer of 2 to 5, R²s may be different from one another)] (hereinafter referred to as aldol compound (6)), comprising a step of reacting the hydrazide compound (4) with a trifluoroacetophenone compound represented by formula (5) : 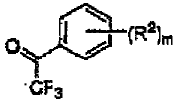 [wherein R² represents a C1-C6 alkyl group optionally having one or more halogen atoms or a halogen atom, and m represents an integer of 0 to 5 (when m is an integer of 2 to 5, R²s may be different from one another)] (hereinafter referred to as trifluoroacetophenone compound (5)).
[6] A method for producing an enone compound represented by formula (7) : 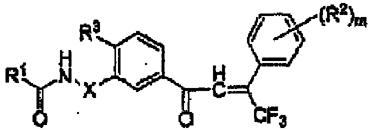 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, R² represents a C1-C6 alkyl group optionally having one or more halogen atoms or a halogen atom, R³ represents a halogen atom or a hydrogen atom, X represents NH or NCH₃, and m represents an integer of 0 to 5 (when m is an integer of 2 to 5, R²s may be different from one another)] (hereinafter referred to as enone compound (7)), comprising a steps of reacting the aldol compound (6) with the acid anhydride compound (2) and then reacting the resultant with a base.
[7] A method for producing an isoxazoline compound represented by formula (8): 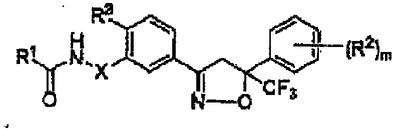 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, R² represents a C1-C6 alkyl group optionally having one or more halogen atoms or a halogen atom, R³ represents a halogen atom or a hydrogen atom, X represents NH or NCH₃, and m represents an integer of 0 to 5 (when m is an integer of 2 to 5, R²s may be different from one another)] (hereinafter referred to as isoxazoline compound (8)), comprising a steps of reacting the hydrazine compound (1) with the acid anhydride compound (2) or the acid chloride (3) to produce the hydrazide compound (4),
   reacting the obtained hydrazide compound (4) with the trifluoroacetophenone compound (5) in the presence of a base to produce the aldol compound (6),
   reacting the obtained aldol compound (6) with the acid anhydride compound (2) and then reacting the resultant with a base to produce the enone compound (7), and
   reacting the obtained enone compound (6) with hydroxylamine.
[8] An enone compound represented by formula (7) : 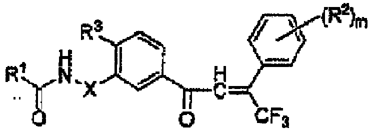 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, R² represents a C1-C6 alkyl group optionally having one or more halogen atoms or a halogen atom, R³ represents a halogen atom or a hydrogen atom, X represents NH or NCH₃, and m represents an integer of 0 to 5 (when m is an integer of 2 to 5, R²s may be different from one another)].
[9] A method for producing the isoxazoline compound (8), comprising a step of reacting the enone compound (7) with hydroxylamine.

### EFFECT OF THE INVENTION

According to the present invention, a new method for producing the isoxazoline compound (8) and the like can be provided.

### MODE FOR CARRYING OUT THE INTENTION

Examples of the "C1-C12 alkyl group optionally having one or more halogen atoms" represented by R¹ include a methyl group, an ethyl group, a propyl group, an isopropyl group, a butyl group, a tert-butyl group, an isobutyl group, a butyl group, a pentyl group, a hexyl group, a dodecyl group, a trifluoromethyl group, and a 3,3,3-trifluoropropyl group.

Examples of the "C3-C12 cycloalkyl group optionally having one or more halogen atoms" represented by R¹ include a cyclopropyl group, a cyclobutyl group, a cyclopentyl group, a cyclohexyl group, and a cycloheptyl group.

Examples of the "C1-C12 alkoxy group optionally having one or more halogen atoms" represented by R¹ include a methoxy group, an methoxy group, a propoxy group, an isopropoxy group, and a tert-butoxy group.

Examples of the "halogewatom" represented by R² include a fluorine atom, a chlorine atom, a bromine atom, and an iodine atoms.

Examples of the "C1-C6 alkyl group optionally having one or more halogen atoms" represented by R² include a methyl group and a trifluoromethyl group.

Examples of the "halogen atom" represented by R³ include a fluorine atom, a chlorine atom, a bromine atom, and an iodine atom.

In the production of the hydrazide compound (4) from the hydrazine compound (1), the hydrazide compound (4) can be produced by a reaction of the hydrazine compound (1) with the acid anhydride compound (2) or the acid chloride (3).

The amount of the acid anhydride compound (2) or the acid chloride (3) used in the reaction is usually 1 to 10 mol, based on 1 mol of the hydrazine compound (1).

The reaction can be carried out also in the presence of a solvent. Examples of the solvent used in the reaction include ethers such as tetrahydrofuran, hydrocarbons such as toluene and halogenated hydrocarbons such as chloroform, and these solvents can be used alone or in combination of two or more thereof.

The amount of the solvent used when the reaction is carried out in the presence of a solvent is 100 L or less, based on 1 mol of the hydrazine compound (1).

The reaction can be carried out also in the presence of a base. Examples of the base used in the reaction include pyridine, triethylamine, and diazabicycloundecene (1,8-diazabicyclo[5.4.0]-7-undecene).

The amount of the base used when the reaction is carried out in the presence of a base is usually 10 mol or less, based on 1 mol of the hydrazine compound (1).

The reaction temperature of the reaction is usually in the range of -20 to 100°C or not more than the boiling point of the solvent used in the reaction.

The reaction time of the reaction is usually in the range of 0.1 to 100 hours.

Thus, the hydrazide compound (4) can be produced. Further, it is also possible to purify the obtained hydrazide compound by means such as extraction by separation, filtration, recrystallization or column chromatography, as necessary.

In the production of the aldol compound (6) from the hydrazide compound (4) and the trifluoroacetophenone compound (5), the aldol compound (6) is produced by a reaction of the hydrazide compound (4) with the trifluoroacetophenone compound (5).

The amount of the trifluoroacetophenone compound. (5) used in the reaction is usually 0.1 to 10 mol, based on 1 mol of the hydrazine compound (4).

The reaction is usually carried out in the presence of a base.

Examples of the base used in the reaction include organic bases such as diazabicycloundecene and 1,5-diazablcyclo[4.3.0]-5-nonene. These bases can be used alone or in combination of two or more thereof.

The amount of the base used in the reaction is usually 0.1 mol to 10 mol, based on 1 mol of the hydrazide compound (4).

The reaction is usually carried out in the presence of a solvent. Examples of the solvent used in the reaction include ethers such as tetrahydrofuran and hydrocarbons such as toluene, and these solvents can be used alone or in combination of two or more thereof.

The amount of the solvent used in the reaction is 100 L or less, based on 1 mol of the hydrazine compound (4).

The reaction temperature of the reaction is usually in the range of -20 to 200°C or not more than the boiling point of the solvent used in the reaction.

The reaction time of the reaction is usually in the range of 0.1 to 100 hours.

Thus, the aldol compound (6) can be produced. Further, it is also possible to purify the obtained aldol compound by means such as extraction by separation, filtration, recrystallization or column chromatography, as necessary.

In the production of the enone compound (7) from the aldol compound (6), the enone compound (7) can be produced by a reaction of the aldol compound (6) with the acid anhydride compound (2) and then with a base.

The reaction is usually divided into Step 1 for reacting the aldol compound (6) with the acid anhydride compound (2), and Step 2 for reacting the resultant with a base after Step 1.

First, Step 1 will be described.

The amount of the acid anhydride compound (2) used in Step 1 is usually 1 to 10 mol, based on 1 mol of the aldol compound (6).

The step is usually carried out in the presence of a base.

Examples of the base used in the step include organic bases such as triethylamine, pyridine, and N,N-dimethyl-4-aminopyridine. These bases can be used alone or in combination of two or more thereof.

The amount of the base used in the step is usually 0.1 to 10 mol, based on 1 mol of the aldol compound (6).

The step is usually carried out in the presence of a solvent. Examples of the solvent used in the step include ethers such as tetrahydrofuran, hydrocarbons such as toluene and halogenated hydrocarbons such as chloroform, and these solvents can be used alone or in combination of two or more thereof.

The amount of the solvent used in the step is 100 L or less, based on 1 mol of the aldol compound (6).

The reaction temperature of the step is usually in the range of -20 to 200°C or not more than the boiling point of the solvent used in the reaction.

The reaction time of the step is usually in the range of 0.1 to 100 hours.

Next, Step 2 will be described.

Step 2 is usually carried out in the presence of a solvent. Examples of the solvent used in Step 2 include methanol.

Examples of the base used in the step include alkali met al hydroxides, alkali metal alkoxides such as sodium methoxide, and alkali metal carbonates.

The amount of the base used in the step is usually 0.01 to 10 mol, based on 1 mol of the aldol compound (6).

The step is usually carried out in the presence of a solvent. Examples of the solvent used in the step include alcohols such as methanol.

The amount of the solvent used in the step is 100 L or less, based on 1 mol of the aldol compound (6).

The reaction temperature of the step is usually in the range of -20 to 100°C or not more than the boiling point of the solvent used in the reaction.

The reaction time of the step is usually in the range of 1 minute to 100 hours.

Thus, the enone compound (7) can be produced. Further, it is also possible to purify the obtained enone compound by means such as extraction by separation, filtration, recrystallization or column chromatography, as necessary.

In the production of the isoxazoline compound (8) from the enone compound (7), the isoxazoline compound (8) can be produced by a reaction of the enone compound (7) with hydroxylamine.

The reaction is usually carried out in the presence of a base.

Examples of the base used in the reaction include metal hydroxides such as sodium hydroxide and organic bases such as diazabicycloundecene. These bases can be used alone or in combination of two or more thereof.

The amount of the base used in the reaction is usually 0.01 to 10 mol, based on 1 mol of the enone compound (7).

The reaction is usually carried out in the presence of a solvent. Examples of the solvent used in the reaction include hydrocarbons such as toluene, ethers such as methyl tert-butyl ether, and water, and these solvents can be used alone or in combination of two or more thereof.

The amount of the solvent used in the reaction is 100 L or less, based on 1 mol of the enone compound (7).

As the hydroxylamine used in the reaction, hydroxylamine in the form of an aqueous solution or a salt such as hydroxylamine hydrochloride can be used. These can be used alone or in combination of two or more thereof.

The amount of the hydroxylamine used in the reaction is usually 1 to 10 mol, based on 1 mol of the enone compound (7).

The reaction can be carried out also in the presence of a phase transfer catalyst. Examples of the phase transfer catalyst used in the reaction include tetrabutylammonium bromide and tetraoctylammonium chloride. These phase transfer catalysts can be used alone or in combination of two or more thereof.

The amount of the phase transfer catalyst used in the reaction is usually 0.001 to 1 mol, based on 1 mol of the enone compound (7).

The reaction temperature of the reaction is usually in the range of -20 to 200°C or the boiling point of the solvent used in the reaction.

The reaction time of the reaction is usually in the range of 0.1 to 100 hours.

Thus, the isoxazoline compound (8) can be produced. Further, it is also possible to purify the obtained isoxazoline compound by means such as extraction by separation, filtration, recrystallization or column chromatography, as necessary.

Some of the hydrazine compounds (1) are commercially available, and other compounds can be produced when X is NH, for example, according to the method described in the Fourth Series of Experimental Chemistry, (Maruzen), Vol. 20, p. 338.

Some of the trifluoroacetophenone compounds (5) are commercially available, and other compounds can be produced, for example, according to the method described in Canadian Journal of Chemistry 58, 2491 (1980) or Angewandte Chemie International Edition 37, 820 (1998).

Specific examples of the hydrazide compound (4) are shown below. The present invention is not limited to these examples.

Hydrazide compounds represented by formula (4) : 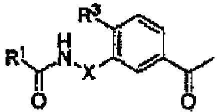 wherein R¹ and R³ represent the combination described in Table 1 bellow.

**[Table 1]**

| No. | R¹ | R³ | X |
|---|---|---|---|
| 1 | CH₃ | Cl | NH |
| 2 | CH₂CH₃ | Cl | NH |
| 3 | CH₂CH₂CH₃ | Cl | NH |
| 4 | CH(CH₃)₂ | Cl | NH |
| 5 | C(CH₃)₃ | Cl | NH |
| 6 | CH₂CH(CH₃)₂ | Cl | NH |
| 7 | CF₃ | Cl | NH |
| 8 | CH₂CH₂CF₃ | Cl | NH |
| 9 | OCH₃ | Cl | NH |
| 10 | OCH₂CH₃ | Cl | NH |
| 11 | OCH(CH₃)₂ | Cl | NH |
| 12 | OC(CH₃)₃ | Cl | NH |
| 13 | CH₃ | H | NH |
| 14 | CH₃ | F | NH |
| 15 | CH₃ | Br | NH |
| 16 | Cyclopropyl | Cl | NH |
| 17 | Cyclobutyl | Cl | NH |
| 18 | Cyclopentyl | Cl | NH |
| 19 | Cyclohexyl | Cl | NH |
| 20 | Cycloheptyl | Cl | NH |
| 21 | (CH₂)₃CH₃ | Cl | NH |
| 22 | (CH₂)₄CH₃ | Cl | NH |
| 23 | (CH₂)₅CH₃ | Cl | NH |
| 24 | (CH₂)₁₁CH₃ | Cl | NH |
| 25 | CH₃ | Cl | NH |
| 26 | CH₃ | Cl | NH |

Specific examples of the aldol compound (6) are shown below.

Aldol compounds represented by formula (6): 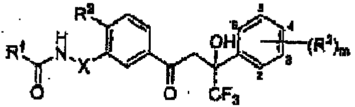 wherein R¹, (R²)ₘ, and R³ represent the combination described in Table 2 below.

**[Table 2]**

| No. | R¹ | (R²)ₘ | R³ | X |
|---|---|---|---|---|
| 1 | CH₃ | 3-Cl, 5-Cl | Cl | NH |
| 2 | CH₂CH₃ | 3-Cl, 5-Cl | Cl | NH |
| 3 | CH₂CH₂CH₃ | 3-Cl, 5-Cl | Cl | NH |
| 4 | CH(CH₃)₂ | 3-Cl, 5-Cl | Cl | NH |
| 5 | C(CH₃)₃ | 3-Cl, 5-Cl | Cl | NH |
| 6 | CH₂CH(CH₃)₂ | 3-Cl, 5-Cl | Cl | NH |
| 7 | CF₃ | 3-Cl, 5-Cl | Cl | NH |
| 8 | CH₂CH₂CF₃ | 3-Cl, 5-Cl | Cl | NH |
| 9 | OCH₃ | 3-Cl, 5-Cl | Cl | NH |
| 10 | OCH₂CH₃ | 3-Cl, 5-Cl | Cl | NH |
| 11 | OCH(CH₃)₂ | 3-Cl, 5-Cl | Cl | NH |
| 12 | OC(CH₃)₃ | 3-Cl, 5-Cl | Cl | NH |
| 13 | CH₃ | 3-Cl, 5-Cl | H | NH |
| 14 | CH₃ | 3-Cl, 5-Cl | F | NH |
| 15 | CH₃ | 3-Cl, 5-Cl | Br | NH |
| 16 | Cyclopropyl | 3-Cl, 5-Cl | Cl | NH |
| 17 | Cyclobutyl | 3-Cl, 5-Cl | Cl | NH |
| 18 | Cyclopentyl | 3-Cl, 5-Cl | Cl | NH |
| 19 | Cyclohexyl | 3-Cl, 5-Cl | Cl | NH |
| 20 | Cycloheptyl | 3-Cl, 5-Cl | Cl | NH |
| 21 | (CH₂)₃CH₃ | 3-Cl, 5-Cl | Cl | NH |
| 22 | (CH₂)₄CH₃ | 3-Cl, 5-Cl | Cl | NH |
| 23 | (CH₂)₅CH₃ | 3-Cl, 5-Cl | Cl | NH |
| 24 | (CH₂)₁₁CH₃ | 3-Cl, 5-Cl | Cl | NH |
| 25 | CH₃ | 3-CF₃, 5-CF₃ | Cl | NH |
| 26 | CH₃ | 3-Cl, 4-Cl, 5-Cl | Cl | NH |

Specific examples of the enone compound (7) are shown below.

Enone compounds represented by formula (7): 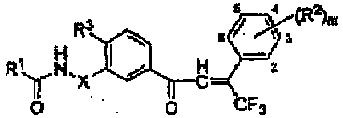 wherein R¹, (R²)ₘ, and R³ represent the combination described in Table 3 below.

**[Table 3]**

| No. | R¹ | (R²)ₙ | R³ | X |
|---|---|---|---|---|
| 1 | CH₃ | 3-Cl, 5-Cl | Cl | NH |
| 2 | CH₂CH₃ | 3-Cl, 5-Cl | Cl | NH |
| 3 | CH₂CH₂CH₃ | 3-Cl, 5-Cl | Cl | NH |
| 4 | CH(CH₃)₂ | 3-Cl, 5-Cl | Cl | NH |
| 5 | C(CH₃)₃ | 3-Cl, 5-Cl | Cl | NH |
| 6 | CH₂CH(CH₃)₂ | 3-Cl, 5-Cl | Cl | NH |
| 7 | CF₃ | 3-Cl, 5-Cl | Cl | NH |
| 8 | CH₂CH₂CF₃ | 3-Cl, 5-Cl | Cl | NH |
| 9 | OCH₃ | 3-Cl, 5-Cl | Cl | NH |
| 10 | OCH₂CH₃ | 3-Cl, 5-Cl | Cl | NH |
| 11 | OCH(CH₃)₂ | 3-Cl, 5-Cl | Cl | NH |
| 12 | OC(CH₃)₃ | 3-Cl, 5-Cl | Cl | NH |
| 13 | CH₃ | 3-Cl, 5-Cl | H | NH |
| 14 | CH₃ | 3-Cl, 5-Cl | F | NH |
| 15 | CH₃ | 3-Cl, 5-Cl | Br | NH |
| 16 | Cyclopropyl | 3-Cl, 5-Cl | Cl | NH |
| 17 | Cyclobutyl | 3-Cl, 5-Cl | Cl | NH |
| 18 | Cyolopentyl | 3-Cl, 5-Cl | Cl | NH |
| 19 | Cyclohexyl | 3-Cl, 5-Cl | Cl | NH |
| 20 | Cycloheptyl | 3-Cl, 5-Cl | Cl | NH |
| 21 | (CH₂)₃CH₃ | 3-Cl, 5-Cl | Cl | NH |
| 22 | (CH₂)₄CH₃ | 3-Cl, 5-Cl | Cl | NH |
| 23 | (CH₂)₅CH₃ | 3-Cl, 5-Cl | Cl | NH |
| 24 | (CH₂)₁₁CH₃ | 3-Cl, 5-Cl | Cl | NH |
| 25 | CH₃ | 3-CF₃, 5-CF₃ | Cl | NH |
| 26 | CH₃ | 3-Cl, 4-Cl, 5-Cl | Cl | NH |

Here, in (R²)ₘ of the above table, for example, the description of "3-Cl, 5-Cl" means that (R²)ₘ is substituents in the positions 3 and 5, m is 2, and R²s are each a chlorine atom.

### EXAMPLES

Examples of the present invention are shown below, but the present invention is not limited by these examples.

### Example 1

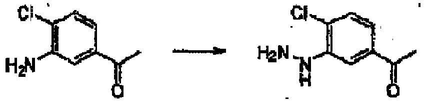

A mixture of 3-acetyl-6-chloroaniline (5.0 g) and concentrated hydrochloric acid (25 ml) was stirred under ice-cooling, and a mixed solution of sodium nitrite (2.1 g) and water (3 ml) was added thereto over 10 minutes. After stirring at room temperature for 30 minutes, a mixture of tin (II) chloride (11.2 g) and concentrated hydrochloric acid (10 ml) was added thereto. After stirring at room temperature for 3 hours, the generated solid was collected by filtration. This solid was washed with hexane to obtain 10 g of a solid. A mixture of the obtained solid (0.5 g) and methyl tert-butyl ether (5 ml) was stirred, and aqueous ammonia (8 ml) was added thereto. The oil layer was separated, and the aqueous layer was extracted four times with methyl tert-butyl ether (5 ml). The oil layers were combined, dried over anhydrous sodium sulfate and concentrated to obtain (3-acetyl-6-chlorophenyl)hydrazine (0.13 g, yield of 48%) as crystals.
¹H-NMR (400 MHz, CDCl₃) δ 2.60 (3H, s), 3.67 (2H, br s), 5.82 (1H, br s), 7.31-7.32 (2H, m), 7.72 (1H, d).

### Example 2

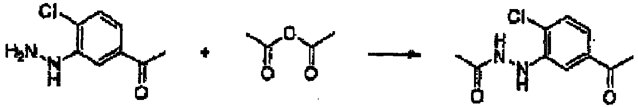

Acetic acid anhydride (0.1 mol) was added to a mixed solution of (3-acetyl-6-chlorophenyl)hydrazine (0.13 g) and chloroform (2 ml). After being left at room temperature for 3 days, the mixture was concentrated. The resulting residue was subjected to silica gel column chromatography (methanol : chloroform =1 : 10) to obtain N'-(5-acetyl-2-chlorophenyl)acetohydrazide (Compound of Table 1-No. 1, 123 mg, yield of 77%) as crystals.

¹H-NMR spectrum was observed as a mixture of rotational isomers, and the peak of the main isomer is herein described. ¹H-NMR (400 MHz, CDCl₃) δ 2.13 (3H, s), 2.56 (3H, s), 6.47 (1H, br d), 7.38-7.41 (3H, m), 7.48 (1H, br d.).

### Example 3

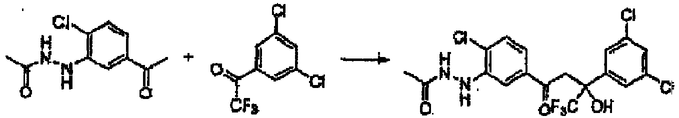

Diazabicycloundecene (22 mg) was added to a mixed solution of N'-(5-acetyl-2'-chlorophenyl)acetohydrazide (103 mg), α,α,α-trifluoro-3,5-dichloroacetophenone (127 mg) and tetrahydrofuran (2 ml). After being left at room temperature for 16 hours, the reaction solution was subjected to silica gel column chromatography to obtain N'-{2-chloro-5-[3-(3,5-dichlorophenyl)-4,4,4-trifluoro-3-hydroxybutanoyl]phenyl} acetohydrazide (Compound of Table 2-No. 1, 97 mg, yield of 45%).

¹H-NMR spectrum was observed as a mixture of rotational isomers, and the peak of the main isomer is herein described. ¹H-NMR (400 MHz, CDCl₃) δ 2.13 (3H, s), 3.63 (1H, d), 3.80 (1H, d), 5.65 (1H, s), 6.54 (1H, br s), 7.23-7.52 (6H, m).

### Example 4

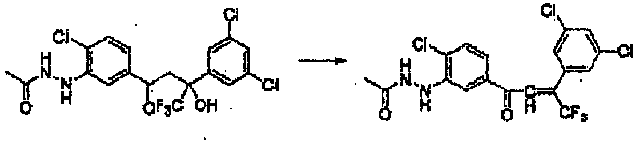

N,N-dimethyl-4-aminopyridine (19 mg) was added to a mixed solution of N'-{2-chloro-5-[3-(3,5-dichlorophenyl)-4,4,4-trifluoro-3-hydroxybutanoyl]phenyl}acetohydrazide (13 mg) and chloroform (0.5 mol) at room temperature, and subsequently acetic acid anhydride (10 mg) was added thereto. After the mixture was left at room temperature for 15 minutes, a saturated sodium bicarbonate solution was added thereto, and the mixture was extracted with methyl tert-butyl ether. The resulting oil layer was concentrated, then diluted with methanol (1 ml), and sodium methoxide (28% methanol solution) (10 mg) was added thereto. After 1 minute, water was added, and the mixture was extracted with methyl tert-butyl ether. The oil layer was concentrated, and the resulting residue was subj ected to silica gel column chromatography [ethyl acetate : hexane = l : 1} to obtain N'-{2-chloro-5-[3-(3,5-dichlorophenyl)-4,4,4-trifluoro-2-butenoyl]phenyl}acetohydrazide (Compound of Table 3-No. 1, 4 mg, yield of 32%).

¹H-NMR spectrum was observed as a mixture derived from rotational isomers or diastereomers of the double bond, and the peak of the main isomer is herein described. ¹H-NMR (400 MHz, CDCl₃) δ 2.12 (3H, s), 3.47 (1H, s), 6.51 (1H, s), 7.15-7.40 (H, m),

### Example 5

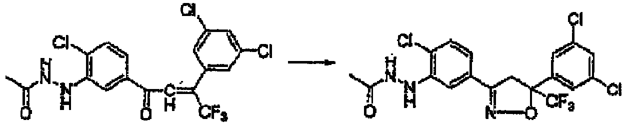

A mixed solution of N'-{2-chloro-5-[3-(3,5-dichlorophenyl)-4,4,4-trifluoro-2-butenoyl]phenyl} acetohydrazide (2 mg), diazabicycloundecene (30 mg) and toluene (1 ml) was stirred, and an aqueous solution (0.2 ml) of hydroxylamine hydrochloride (10 mg) was added thereto at room temperature. After the mixture was left at room temperature for 16 hours, water was added, and the mixture was extracted with methyl tert-butyl ether. The oil layer was concentrated, and the resulting residue was subjected to silica gel column chromatography (ethyl acetate : hexane =1 : 1) to obtain N'-{2-chloro-5- [5-(3,5-dichlorophenyl)-5-(trifluoromethyl)-4,5-dihydroisoxazol-3-yl]phenyl}-acetohydrazide (0.7 mg, yield of 31%).

¹H-NMR spectrum was observed as a mixture of rotational isomers, and the peak of the main isomer is herein described. ¹H-NMR (400 MHz, CDCl₃) δ 2.14 (3H, s), 3.64 (1H), 4.03 (1H, d), 7.26-7.52 (6H, m).

### Example 6

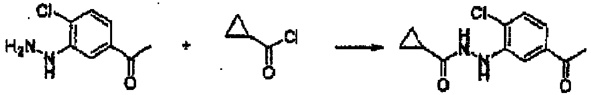

A mixed solution of (3-acetyl-6-chlorophenyl)hydrazine (0.43 g), chloroform (30 ml) and pyridine (1.7 g) was stirred, and added to cyclopropane carboxylic acid chloride (0.58 g) under ice-cooling. After 30 minutes, water was added, and the mixture was extracted with ethyl acetate and dried over sodium sulfate, then concentrated. The resulting residue was subjected to silica gel column chromatography (ethyl acetate : hexane =1 : 2) to obtain N'-(5-acetyl-2-chlorophenyl)cyclopropanecarbohydrazide (Compound of Table 1-No. 16, 123 mg, yield of 56%) as crystals.

¹H-NMR spectrum was observed as a mixture of rotational isomers, and the peak of the main isomer is herein described. ¹H-NMR (400 MHz, CDCl₃) δ 0.87 (2H, m), 1.06 (2H, m), 1.54 (1H, m}, 2.56 (3H, s), 6.55 (1H, br s), 7.34-7.40 (2H, m), 7.52 (1H, s), 7.59 (1H, br,s).

### Example 7

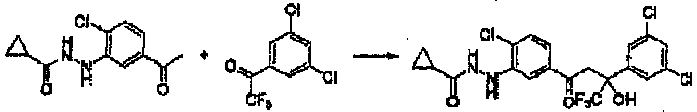

Diazabicycloundecene (14 mg) was added to a mixed solution of N'-(5-acetyl-2-chlorophenyl)cyclopropanecarbohydrazide (231 mg), α,α,α-trifluoro-3,5-dichloroacetophenone (333 mg) and tetrahydrofuran (10 ml). After being left at room temperature for 16 hours, the reaction solution was subjected to silica gel column chromatography to obtain N'-{2-chloro-5-[3-(3,5-dichlorophenyl)-4,4,4-trifluoro-3-hydroxybutanoyl]phenyl} cyclopropanecarbohydrazide (Compound of Table 2-No. 16, 236 mg, yield of 52%).

¹H-NMR spectrum was observed as a mixture of rotational isomers, and the peak of the main isomer is herein described. ¹H-NMR (400 MHz, CDCl₃) δ 0.89 (2H, m), 1.03 (2H, m), 1.52 (1H, m), 3.63 (1H, d), 3.79 (1H, d), 5.69 (1H, s), 6.55 (1H, s), 7.35-7.51 (5H, m), 7.62 (1H, s).

### Example 8

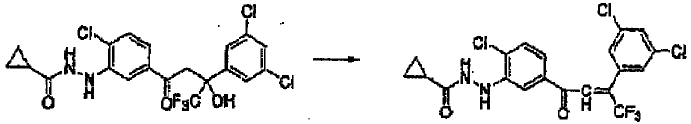

N,N-dimethyl-4-aminopyridine (100 mg) was added to a mixed solution of N'-{2-chloro-5-[3-(3,5-dichlorophenyl)-4,4,4-trifluoro-3-hydroxybutanoyl]phenyl}cyclopropanecarboh ydrazide (16 mg) and chloroform (2 ml) at room temperature, and subsequently acetic acid anhydride (60 mg) was added thereto. After the mixture was left at room temperature for 16 hours, water was added, and the mixture was extracted with methyl tert-butyl ether. The resulting oil layer was concentrated, then diluted with methanol (1 ml), and sodium methoxide (28% methanol solution) (200mg) was added thereto. After 3 minutes, water was added, and the mixture was extracted with methyl tert-butyl ether. The oil layer was concentrated, and the resulting residue was subjected to silica gel column chromatography (ethyl acetate : hexane = 1 : 2} to obtain N'-{2-chloro-5-[3-(3,5-dichlorophenyl)-4,4,4-trifluoro-2-bu tenoyl]phenyl]cyclopropanecarbohydrazide (Compound of Table 3-No. 16, 3.5 mg, yield of 23%).

¹H-NMR spectrum was observed as a mixture derived from rotational isomers or diastereomers of the double bond, and the peak of the main isomer is herein described. ¹H-NMR (400 MHz, CDCl₃) δ 0.92 (2H, m), 1.06 (2H, m), 1.24 (1H, m), 3.23 (1H, s), 6.53 (1H, s), 7.15-7.48 (7H, m).

### Example 9

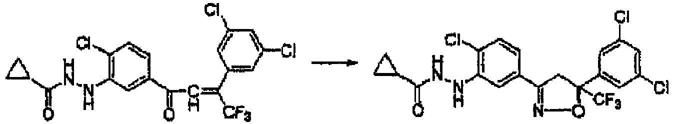

A mixed solution of sodium hydroxide (44 mg) and water (0.5 ml) was stirred at room temperature, and a mixed solution of hydroxylamine hydrochloride (38 mg), N'-{2-chloro-5-[3-(3,5-dichlorophenyl)-4,4,4-trifluoro-2-butenoyl]phenyl} cyclopropanecarbohydrazide (3.2 mg) and methyl tert-butyl ether (0.5 ml), and tetrabutylammonium bromide (3 mg) were sequentially added thereto. After the mixture was left at room temperature for 3 days, water was added, and the mixture was extracted with methyl tert-butyl ether. The oil layer was concentrated, and the resulting residue was subjected to silica gel column chromatography (ethyl acetate : hexane =1 ; 2) to obtain N'-{2-chloro-5-[5-(3,5-dichlorophenyl)-5-(trifluoromethyl)-4,-5-dihydroisoxazol-3-yl]phenyl}-cyclopropanecarbohydrazide (1.5 mg, yield of 45%).

¹H-NMR spectrum was observed as a mixture of rotational isomers, and the peak of the main isomer is herein described. ¹H-NMR (400 MHz, CDCl₃) δ 0.92 (2H, m), 1.07 (2H, m), 1.53 (1H, m), 3.65 (1H, d), 4.04 (1H, d), 6.53 (1H, br s), 6.94-7.49 (7H, m).

## Claims

1. A hydrazide compound represented by formula (4): 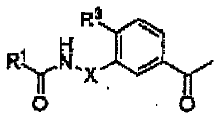 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, R³ represents a halogen atom or a hydrogen atom, and X represents NH or NCH₃].

2. An aldol compound represented by formula (6): 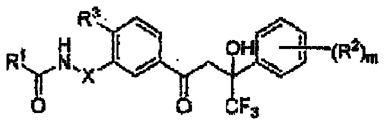 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, R² represents a C1-C6 alkyl group optionally having one or more halogen atoms or a halogen atom, R³ represents a halogen atom or a hydrogen atom, X represents NH or NCH₃, and m represents an integer of 0 to 5 (when m is an integer of 2 to 5, R²s may be different from one another)].

3. (3-Acetyl-6-chlorophenyl)hydrazine.

4. A method for producing a hydrazine compound represented by formula (4): 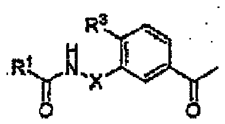 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, R³ represents a halogen atom or a hydrogen atom, and X represents NH or NCH₃], comprising a step of reacting a hydrazine compound represented by formula (1}: 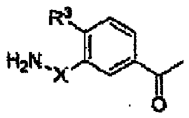 [wherein R³ and X represent the same meanings as above] with an acid anhydride compound represented by formula (2): 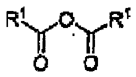 [wherein R¹ represents the same meaning as above] or an acid chloride represented by formula (3): 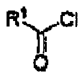 [wherein R¹ represents the same meaning as above].

5. A method for producing an aldol compound represented by formula (6): 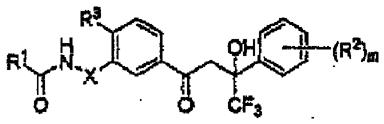 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, R² represents a C1-C6 alkyl group optionally having one or more halogen atoms or a halogen atom, R³ represents a halogen atom or a hydrogen atom, X represents NH or NCH₃, and m represents an integer of 0 to 5 (when m is an integer of 2 to 5, R²s may be different from one another)],
comprising a step of reacting a hydrazide compound represented by formula (4): 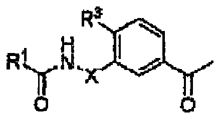 [wherein R¹, R³ and X represent the same meanings as above] with a trifluoroacetophenone compound represented by formula (5): 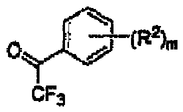 [wherein R² and m represent the same meanings as above].

6. A method for producing an enone compound represented by formula (7): 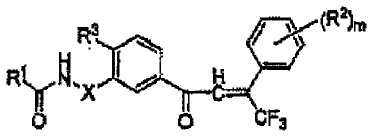 [wherein R¹ represents a C1-C22 alkyl group optionally having one or more halogen atom, a C3-C12 cycloalkyl group optionally having one or more halogen atoms or a C1-C12 alkoxy group optionally having one or more halogen atoms, R² represents a C1-C6 alkyl group optionally having one or more halogen atoms or a halogen atom, R³ represents a halogen atom or a hydrogen atom, X represents NH or NCH₃, and m represents an integer of 0 to 5 (when m is an integer of 2 to 5, R²s may be different from one another)],
comprising a steps of reacting an aldol compound represented by formula (6): 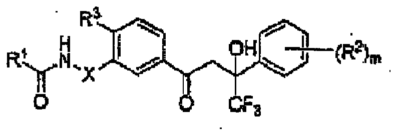 [wherein R¹, R², R³, X and m represent the same meanings as above] with an acid anhydride compound represented by formula (2): 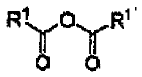 [wherein R¹ represents the same meaning as above] and then reacting the resultant with a base.

7. A method for producing an isoxazoline compound represented by formula (8): 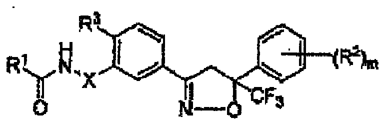 [wherein R¹ represents a C1-C12 alkyl group optionally having one or more halogen atoms, a C3-C12 cycloalkyl group optionally having one or more halogen atoms or a C1-C12 alkoxy group optionally having one or more halogen atoms, R² represents a C1-C6 alkyl group optionally having one or more halogen atoms or a halogen atom, R³ represents a halogen atom or a hydrogen atom, X represents NH or NCH₃, and m represents an integer of 0 to 5 (when m is an integer of 2 to 5, R²s may be different from one another)],
comprising steps of:
reacting a hydrazine compound represented by formula (1): 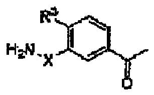 [wherein R³ and X represent the same meanings as above] with an acid anhydride compound represented by formula (2): 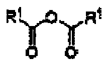 [wherein R¹ represents the same meaning as above] or an acid chloride represented by formula (3): 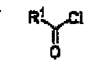 [wherein R¹ represents the same meaning as above] to produce a hydrazide compound represented by formula (4): 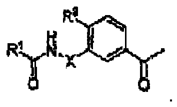 [wherein R³, R³ and X represent the same meanings as above] ; reacting the obtained hydrazide compound represented by the formula (4) with a trifluoroacetophenone compound represented by formula (5): 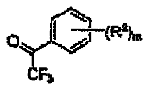 [wherein R² and m represent the same meaning as above] to produce an aldol compound represented by formula (6): 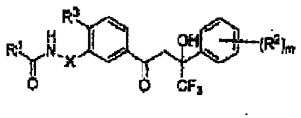 [wherein R¹, R², R³, X and m represent the same meanings as above] ; reacting the obtained aldol compound represented by the formula (6) with the acid anhydride compound represented by the formula (2) and then reacting the resultant with a base to produce an enone compound represented by formula (7): 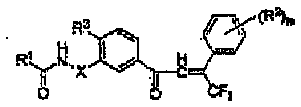 [wherein R¹, R², R³, X and m represent the same meanings as above] ; and reacting the obtained enone compound represented by the formula (7) with hydroxylamine.
